# FASCICULE DE BREVET EUROPEEN

(11) **EP 1 338 289 B1**
(45) Date de publication et mention de la délivrance du brevet: **26.10.2005**
(21) Numéro de dépôt: 03370006.3
(22) Date de dépôt: 29.01.2003
(51) Int. Cl.: A61L 2/24, A61L 11/00

(54) **Dispositif de traitement d'effluents liquides issus de laboratoires d'analyses médicales et son procédé d'utilisation**
Vorrichtung zur Behandlung von flüssigen Abfällen von medizinischen Analyselaboratorien und Verfahren für ihre Verwendung
Device for treating liquid effluents from medical analysis laboratories and process using the same

(30) Priorité: 31.01.2002 FR 0201154
(43) Date de publication de la demande: 27.08.2003
(73) Titulaire: Nisse, SARL, 59553 Cuincy (FR)
(72) Inventeur: Nisse, Bernard, 59553 Lauwin Planque (FR)
(74) Mandataire: Hennion, Jean-Claude

(56) Documents cités:
- EP-A- 0 634 178
- DE-C- 19 827 404
- FR-A- 2 762 789
- FR-A- 2 772 276
- US-A- 5 914 047

## Description

L'invention se rapporte à un dispositif de décontamination des effluents liquides issus de laboratoires d'analyses médicales ou d'établissements hospitaliers.

De nombreux travaux ont été réalisés ces dernières années sur les filières et les modes d'élimination des déchets liquides issus de laboratoires d'analyses médicales ou d'hôpitaux.

Ces travaux démontrent l'intérêt que les professionnels de santé ainsi que les pouvoirs publics attachent à ce sujet.

Cette prise de conscience est apparue de façon concomitante avec de nouveaux micro-organismes (VIH, bactéries multi-résistantes et prions).

De nombreuses technologies employées en centre hospitalier (hémodialyses, radiologies, laboratoires d'analyse) engendrent des effluents liquides qui sont potentiellement chargés en micro-organismes, dont certains sont multi-résistant.

Parmi les sources de contamination de ces effluents, on peut citer :
- les excrétions et déjections des patients atteints de maladies à déclaration obligatoire de type entérique (salmonellose, shigellose, choléra, botulisme) ;
- pièces anatomiques ou tissus provenant de patients infectés par les hépatites, la tuberculose, la maladie de Creuzfeld Jacob, le sida ;
- pour les effluents mixtes chimico-biologiques, composés de liquides biologiques mélangés à des réactifs chimiques lors des techniques d'analyses manuelles ou automatisées, on peut citer notamment les substances géno-toxiques, cancérogènes, mutagènes et/ou toxiques pour la reproduction, utilisées plus particulièrement dans les laboratoires de recherches bio-médicales.

On peut également citer les colorants utilisés pour les diverses colorations en laboratoires (gram, ziehl, may-grunwald, giemsa) ou bien encore les dérivés de cyanure conventionnellement neutralisés par oxydation en cyanates avant rejet en égout.

Les laboratoires d'analyses médicales ou les laboratoires de recherches bio-médicales sont ainsi amenés à étudier et analyser des produits extrêmement variés (sang, urine, selles, liquides gastrique, aspiration trachéo-bronchique, liquide d'épanchement péritonéal ou pleurale, liquide de drainage ou d'irrigation), les effluents issus de ces analyses comprenant des liquides biologiques mélangés à des réactifs chimiques, les produits chimiques utilisés pouvant avoir en eux-mêmes un effet toxique, allergisant, mutagène, tératogène ou géno-toxique, les liquides biologiques analysés pouvant être en eux-mêmes contaminés par des bactéries multi-résistantes, des virus ou des prions.

L'arrêté du 26 novembre 1999 du ministère des affaires sociales, de la santé et de la ville, relatif à la bonne exécution des analyses de biologie médicale contient des règles auxquelles doivent se conformer les laboratoires de biologie médicale.

L'article 6.1 précise que l'élimination des déchets doit être conforme à la législation en vigueur et doit être conduite de manière à ne pas compromettre la santé du personnel du laboratoire et celui chargé de la collecte des déchets et ne pas polluer l'environnement.

On connaît déjà dans l'art antérieur divers décontaminants pour liquides biologiques.

Ainsi, par exemple, le décontaminant SANYTEX de la société ROCHEX présente une action virucide pour les orthopox-virus (virus enveloppés à ADN), mais aussi le HIV, les adénovirus, (virus non enveloppés à ADN).

Ce décontaminant est un composé d'ammonium quaternaire et de glutaraldéhyde. Le glutaraldéhyde est utilisé conventionnellement pour la désinfection des matériels médicaux chirurgicaux tels qu'endoscopes et fibroscopes.

Un grand nombre de nettoyants désinfectants sont connus dans l'art antérieur.

Ainsi, par exemple, la société ALCO FRANCE commercialise des nettoyants désinfectants sous la dénomination IDOS.

Il s'agit de chlorure de didécyldiméthylammonium, mélangé à des tensioactifs cationiques, des sels alcalins de sodium, du nonylphénol oxyéthylène, des tensioactifs non ioniques et des sels d'EDTA.

Ce produit IDOS DN, lorsque mis en solution à température ambiante, présente une efficacité certaine contre le virus de l'hépatite B et le HIV.
On connaît par ailleurs de nombreuses conceptions de dispositifs pour la stérilisation ou la décontamination d'effluents liquides issus de milieux hospitaliers.

En général, ces dispositifs de l'état de la technique comprennent :
- un réservoir de réception et de stockage des effluents liquides ;
- un moyen de mélange d'une quantité déterminée d'un agent de stérilisation avec les effluents ;
- un moyen de surveillance de la quantité d'effluents reçus par le réservoir de stockage ;
- un moyen de détection d'un seuil déterminé pour la commande du moyen de mélange d'une quantité déterminée d'agents de stérilisation avec lesdits effluents ;
- un moyen d'évacuation des effluents traités ;
- un moyen chronométrique de commande du moyen d'évacuation des effluents traités.

Le document US-A-5 914 047 décrit un dispositif pour la décontamination d'effluents liquides d'origine médicale comprenant :
- une chambre d'aspiration 44a,44b comprenant un réservoir 46a,46b et des moyens d'aspiration d'un effluent liquide (tube d'aspiration 50, pompe à vide 58) ;
- une cuve de liquide désinfectant 80 ;
- un mélangeur 76 ;
- des moyens de pompage 104 de l'effluent de la cuve 46a,46b vers le mélangeur 76 et du liquide désinfectant de la cuve 80 vers le mélangeur 76 ;
- une cuve de stockage temporaire 30 du mélange effluent liquide/désinfectant.

Ce dispositif se caractérise par le fait que le liquide désinfectant et l'effluent à traiter sont envoyés en même temps vers le mélangeur 76 au moyen de la pompe 104.

Les dispositifs connus dans l'art antérieur exigent donc la mise en oeuvre d'un grand nombre de moyens spécifiques :
pour la détection d'une quantité très déterminée d'effluents dans un réservoir ;
   - pour le dosage d'un agent stérilisant ;
   - pour le mélange de l'agent stérilisant avec l'effluent à traiter.

D'autres dispositifs de décontamination d'effluents liquides sont adaptés pour le traitement de la phase liquide de substances contaminées comprenant également une phase solide. Un tel dispositif de décontamination est décrit dans le document DE 198 27 404 C1. Ce dispositif comprend :
- un système de transport des effluents liquides comportant une unité d'entrée 4 desdits effluents liquides ;
- une chambre de traitement 2 comportant une zone de collecte 7;
- un récipient collecteur 15 de l'effluent ;
- au moins une conduite 13 d'effluent reliant la zone de collecte 7 du récipient 15, de telle sorte que l'effluent puisse être amené de la zone de collecte 7 dans le récipient collecteur 15, et ramené de ce dernier dans la chambre de traitement 2.

De manière caractéristique, la décontamination des effluents liquides est assurée par un traitement thermique à haute température.

L'invention vise à obtenir un dispositif pour la stérilisation ou la décontamination d'effluents liquides tels que notamment ceux issus d'automates d'analyses, de laboratoires d'analyses médicales, ce dispositif nécessitant la mise en oeuvre de peu de moyens, le fonctionnement de ce dispositif étant simple, sûr et fiable.

L'invention vise également à obtenir un dispositif permettant le traitement des effluents au fur et à mesure de leur production.

A ces fins, l'invention se rapporte, selon un premier aspect à un dispositif de décontamination des effluents liquides issus de laboratoires d'analyses médicales ou d'établissements hospitaliers, caractérisé en ce qu'il comprend deux cuves, à savoir une première cuve de transfert dans laquelle les effluents à traiter sont versés pour y être ensuite transférés dans une deuxième cuve de décontamination dans laquelle les effluents à traiter sont mélangés à un produit décontaminant, les deux cuves étant pourvues de détecteurs de niveau ou sondes, le dispositif étant pourvu de moyens de dosage permettant d'apporter un flux prédéterminé de produit décontaminant dans le flux de déversement de la cuve de transfert vers la cuve de décontamination, le dispositif comprenant une unité centrale de commande commandant :
- une première pompe de déversement des effluents de la première cuve vers la deuxième cuve ;
- une deuxième pompe de déversement du mélange des effluents traités contenus dans la deuxième cuve, vers la sortie d'évacuation telle qu'un tout à l'égout ou un poste de traitement ultérieur,
les détecteurs de niveau adressant des informations à l'unité centrale de commande qui, au vu de ces informations, commande les pompes et les moyens de dosage.

Selon diverses réalisations, le dispositif présente les caractères suivants, éventuellement combinés :
- il comprend en outre une troisième cuve de réserve en produit décontaminant et un moyen de pompage dudit produit décontaminant pour l'amener vers le moyen de dosage pour son mélange avec les effluents à traiter ;
- il comprend des détecteurs de niveau pour le produit décontaminant contenu dans la troisième cuve, ces détecteurs de niveau étant reliés à l'unité centrale de commande ;
- les détecteurs de niveau comprennent une sonde niveau haut, une sonde commune et une sonde niveau bas, les niveaux haut et bas étant prédéterminés pour chaque cuve ;
- les pompes sont immergées pour un gain de place, elles peuvent aussi être à l'extérieur ;
- les pompes sont de type centrifuge ou à membrane ;
- le moyen de pompage du produit décontaminant depuis la troisième cuve comprend une pompe Venturi, péristaltique ou à membrane ;
- il comprend des moyens de vidange manuelle des cuves tels que notamment des robinets disposés en partie basse de ces cuves ;
- il comprend en outre un réservoir formant chambre de décompression apte à assurer la condensation de brouillards tels que ceux issus d'appareils d'hématologie, ces brouillards condensés étant ensuite amenés dans la première cuve de traitement d'effluents liquides ;

L'invention se rapporte, selon un deuxième aspect, à un procédé d'utilisation d'un dispositif tel que présenté ci-dessus, ce procédé comprenant :
- une étape de remplissage de la première cuve en effluents à traiter ;
- la mise en route de la première pompe de cette première cuve, dès qu'un niveau haut prédéterminé est décelé par les détecteurs de niveau de cette première cuve ;
- le mélange en proportion déterminé d'un flux d'effluents issus de la première cuve à un flux de produit décontaminant ;
- le transfert de ce mélange dans la cuve de décontamination, jusqu'à détection d'un niveau haut prédéterminé dans cette deuxième cuve et/ou détection d'un manque d'effluents.

Selon une caractéristique, le procédé comprend en outre une étape de maintien du mélange effluents en cours de traitement / produit décontaminant, pendant un temps réglable, dans la deuxième cuve.

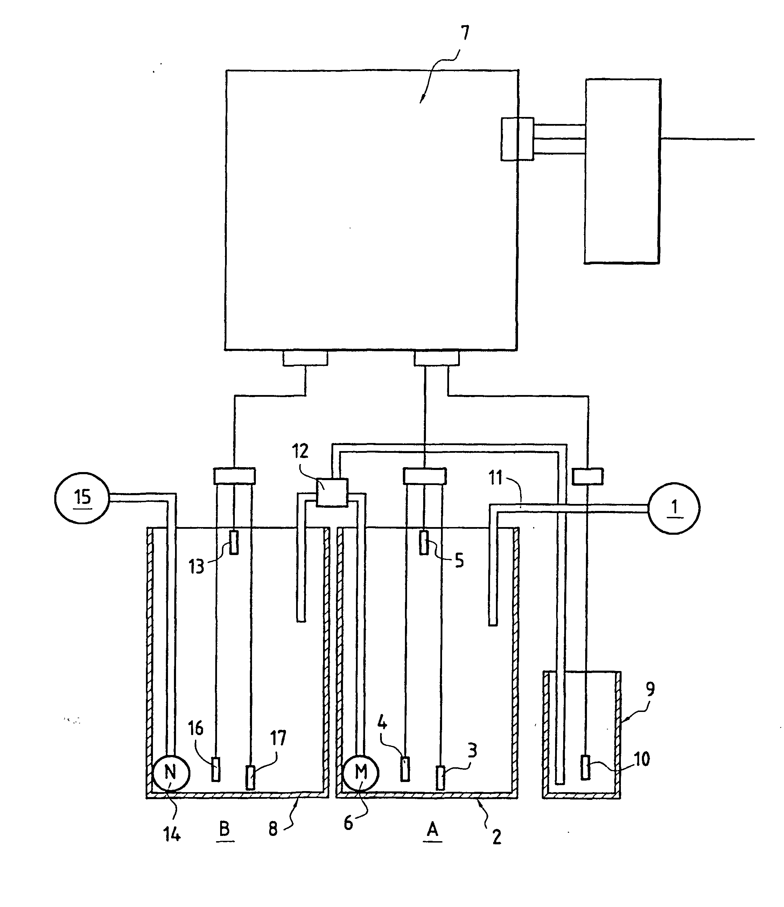

D'autres objets et avantages de l'invention apparaîtront au cours de la description suivante de modes de réalisation, description qui va être effectuée en se référant au dessin unique annexé schématisant les différents composants d'un dispositif selon l'invention.

A fin de clarté, le dispositif schématisé sur la figure annexée va être décrit en suivant le circuit de traitement des effluents.

Les effluents à traiter 1 sont amenés dans une première cuve 2 dite de transfert.

Cette cuve de transfert 2 est pourvue de trois sondes, à savoir : une sonde commune 3, une sonde niveau bas 4 et une sonde niveau haut 5.

Dans la cuve de transfert 2 est placée une pompe 6, immergée ou non, de type centrifuge ou à membrane.

Dès que la sonde niveau haut 5 détecte que la cuve de transfert 2 est remplie jusqu'à un niveau haut prédéterminé, une carte électronique 7 commande la pompe 6 qui vide la cuve de transfert 2 en transférant le contenu de cette cuve de transfert 2 vers une cuve de décontamination 8.

Durant ce transfert, les effluents à décontaminer 1 reçoivent un gradient de décontaminant.

A cette fin, le dispositif comprend une troisième cuve 9 contenant un produit décontaminant tel que par exemple IDOS DN tel que présenté auparavant.

Cette troisième cuve 9 est pourvue d'une sonde de détection du niveau bas 10 assurant la détection d'un éventuel manque de réactif dans cette troisième cuve 9.

Une canne d'aspiration de produit décontaminant amène celui-ci jusque un dispositif doseur 12 disposé en aval de la pompe 6.

Ce dispositif doseur 12 assure le mélange dans les effluents à traiter 1 issus de la cuve de transfert 2 d'une quantité pré-déterminée de produit décontaminant issu de la troisième cuve 9.

Dans un mode de réalisation, une pompe (non représentée) vient prélever le produit décontaminant contenu dans la troisième cuve 9, cette pompe étant de type Venturi, péristaltique ou à membrane ou tout autre moyen apte à amener en aval de la pompe 6 un gradient de produit décontaminant proportionnel au volume transféré de la première cuve 2 vers la cuve de décontamination 8.

La pompe de transfert 6 est arrêtée si la sonde niveau bas 4 de la cuve de transfert 2 détecte un manque d'effluents 1 ou lorsque la sonde niveau haut 13 de la cuve de décontamination 8 détecte que cette cuve est pleine.

Ainsi, le remplissage de la cuve de décontamination 8 peut être effectué en plusieurs fois, par des parties de volume issues de la cuve de transfert 2, en fonction de la hauteur d'entrée des effluents 1 dans la cuve de transfert 2 et de la hauteur des sondes de détection placées dans les cuves 2,8.

La carte électronique 7 impose au dispositif un temps d'incubation et de stérilisation : le produit décontaminant mélangé aux effluents traités 1 est placé dans la cuve de décontamination 8 pendant un temps d'incubation et de stérilisation pré-déterminé, par exemple de trente minutes.

Lorsque cette incubation est terminée, la carte électronique 7 autorise la mise en fonction de la pompe 14 telle qu'une pompe immergée de type centrifuge ou à membrane, cette pompe 14 de la cuve de décontamination 8 assurant le rejet des effluents traités 15, par exemple vers le tout à l'égout.

Ainsi que le comprendra l'homme du métier, le cycle de traitement est continu de sorte que pendant l'incubation dans la cuve de décontamination 8, la cuve de transfert 2 peut être remplie d'effluents 1 à décontaminer.

Lorsque le dispositif est utilisé en hématologie, les effluents parviennent avantageusement dans un premier petit réservoir avant d'entrer dans la cuve de transfert 2, ce petit réservoir formant chambre de décompression.

En effet, les appareils d'hématologie vaporisent ou conduisent facilement à la création d'un brouillard.

Le petit réservoir (non représenté) permet de condenser ce brouillard et de faire couler au fur et à mesure les effluents contenus dans la cuve de transfert 2.

Le dispositif peut être installé en aval de tout système d'analyses non pourvu d'un process de décontamination.

Le dispositif selon l'invention permet d'automatiser cette décontamination.

Le dispositif comprend diverses alarmes visuelles et/ou sonores, de type connu en soi, indiquant à l'opérateur un fonctionnement anormal de l'appareil tel que par exemple manque de réactifs de décontamination dans la cuve 9.

Les deux cuves 2,8 sont pourvues en partie basse d'un robinet (non représenté) permettant la vidange manuelle en cas d'intervention, par suite d'un défaut de fonctionnement.

Ainsi que le comprendra l'homme du métier, les cuves 2,8 sont de structure générale sensiblement identique et comprennent toutes deux trois sondes 3,4,5 ; 13,16,17, ces cuves 2,8 pourront être de volume sensiblement identique ou non.

## Revendications

1. Dispositif de décontamination des effluents liquides issus de laboratoires d'analyses médicales ou d'établissements hospitaliers, comprenant trois cuves, à savoir, une première cuve (2) de transfert dans laquelle les effluents à traiter sont versés pour y être ensuite transférés dans une deuxième cuve (8) de décontamination dans laquelle les effluents à traiter sont mélangés à un produit décontaminant, les deux cuves (2,8) étant pourvues de détecteurs de niveau ou sondes (3,4,5 ; 13,16,17) une troisième cuve (9) de réserve en produit décontaminant ; le dispositif étant pourvu de moyens de dosage (12) permettant d'apporter un flux prédéterminé de produit décontaminant dans le flux de déversement de la cuve de transfert (2) vers la cuve de décontamination (8) le dispositif comprenant une unité centrale de commande (7) commandant :
- une première pompe (6) de déversement des effluents de la première cuve (2) vers la deuxième cuve (8) ;
- une deuxième pompe (14) de déversement du mélange des effluents traités contenus dans la deuxième cuve (8), vers la sortie d'évacuation telle qu'un tout à l'égout ou un poste de traitement ultérieur,
- les détecteurs de niveau (3,4,5 ; 13,16,17) adressant des informations à l'unité centrale de commande qui, au vu de ces informations, commande les pompes (6,14) et les moyens de dosage (12) ;
ledit dispositif étant **caractérisé en ce qu'**il permet la décontamination des effluents au fur et à mesure de leur production et **en ce qu'**il comprend en outre un réservoir formant chambre de décompression apte à assurer la condensation de brouillards tels que ceux issus d'appareils d'hématologie, ces brouillards condensés étant ensuite amenés dans la première cuve (2) de traitement d'effluents liquides.

2. Dispositif selon la revendication 1, **caractérisé en ce qu'**il comprend un moyen de pompage dudit produit décontaminant pour l'amener vers le moyen de dosage (12) pour son mélange avec les effluents à traiter (1).

3. Dispositif selon la revendication 2, **caractérisé en ce qu'**il comprend des détecteurs de niveau (10,11) pour le produit décontaminant contenu dans la troisième cuve (9), ces détecteurs de niveau (10,11) étant reliés à l'unité centrale de commande (7).

4. Dispositif selon l'une quelconque des revendications 1 à 3, **caractérisé en ce que** les détecteurs de niveau comprennent une sonde niveau haut, une sonde commune et une sonde niveau bas, les niveaux haut et bas étant prédéterminés pour chaque cuve (2,8,9).

5. Dispositif selon l'une quelconque des revendications 1 à 4, **caractérisé en ce que** les pompes (6,14) sont immergées.

6. Dispositif selon l'une quelconque des revendications 1 à 5, **caractérisé en ce que** les pompes (6,14) sont de type centrifuge ou à membrane.

7. Dispositif selon l'une quelconque des revendications 1 à 6, **caractérisé en ce que** le moyen de pompage du produit décontaminant depuis la troisième cuve (9) comprend une pompe Venturi, péristaltique ou à membrane.

8. Dispositif selon l'une quelconque des revendications 1 à 7, **caractérisé en ce qu'**il comprend des moyens de vidange manuelle des cuves (2,8,9) tels que notamment des robinets disposés en partie basse de ces cuves (2,8,9).

9. Procédé d'utilisation d'un dispositif tel que présenté dans l'une quelconque des revendications 1 à 8, **caractérisé en ce qu'**il comprend :
- une étape de remplissage de la première cuve (2) en effluents à traiter (1) ;
- la mise en route de la première pompe (6) de cette première cuve (2), dès qu'un niveau haut prédéterminé est décelé par les détecteurs de niveau de cette première cuve (2) ;
- le mélange en proportion déterminé d'un flux d'effluents issus de la première cuve (2) à un flux de produit décontaminant ;
- le transfert de ce mélange dans la cuve de décontamination (8), jusqu'à détection d'un niveau haut prédéterminé dans cette deuxième cuve (8) et/ou détection d'un manque d'effluents.

10. Procédé selon la revendication 9, **caractérisé en ce qu'**il comprend en outre une étape de maintien du mélange effluents en cours de traitement / produit décontaminant, pendant un temps réglable, dans la deuxième cuve (8).

## Claims

1. Device for the decontamination of liquid effluent from medical analysis laboratories or from hospital centres, involving three tanks, namely, a first transfer tank (2) into which the effluent to be treated is discharged, from which it is transferred into a second decontamination tank (8) in which the effluent to be treated is mixed with a decontamination agent, with both tanks (2,8) being equipped with level detectors or probes (3,4,5 ; 13,16,17) and a third tank (9) for storage of decontamination agent; with the device being equipped with means for making controlled additions (12) which allows a pre-determined flow of decontamination agent to be added to the flow being discharged from the transfer tank (2) towards the decontamination tank (8), with the device including a central control unit (7) which controls:
- a first pump (6) for discharging effluent from the first tank (2) towards the second tank (8);
- a second pump (14) for discharging the mixture of treated effluent contained in the second tank (8) towards an outlet, such as a water-born sewage system or a further treatment station,
- the level detectors (3,4,5 ; 13,16,17) which send information to the central control unit, which then uses this information to control the pumps (6, 14) and the means of making controlled additions (12);
with the aforementioned device being **characterised by** the fact that it enables decontamination of effluent to be carried out as and when the effluent is produced and by the fact that it also includes a reservoir which forms a decompression chamber and which is suitable for ensuring that mists such as those discharged from haematology equipment are condensed, with these condensed mists then being led into the first liquid effluent treatment tank (2).

2. Device as described in claim 1, **characterised by** the fact that it includes a means of pumping the aforementioned decontamination agent in order to bring it towards the means for controlled addition (12) so that it may be mixed with the effluent to be treated (1).

3. Device as described in claim 2, **characterised by** the fact that it includes level detectors (10,11) for the decontamination agent contained in the third tank (9), with these level detectors (10,11) being connected to the central control unit (7).

4. Device as described in any of claims 1 to 3 whatsoever, **characterised by** the fact that the level detectors include a high-level probe, a common probe and a low-level probe, with the high- and low-levels being predetermined for each tank (2,8,9).

5. Device as described in any of claims 1 to 4 whatsoever, **characterised by** the fact that the pumps (6,14) are submerged.

6. Device as described in any of claims 1 to 5 whatsoever, **characterised by** the fact that the pumps (6,14) are centrifugal or diaphragm pumps.

7. Device as described in any of claims 1 to 6 whatsoever, **characterised by** the fact that the means for pumping the decontamination agent from the third tank (9) involves a Venturi jet ejector pump, a peristaltic pump or a diaphragm pump.

8. Device as described in any of claims 1 to 7 whatsoever, **characterised by** the fact that it includes means for manually emptying the tanks (2,8,9) such as, in particular, taps arranged at the bottom parts of these tanks (2,8,9).

9. Operating process for a device such as presented in any of claims 1 to 8 whatsoever, **characterised by** the fact that it includes:
- a step for filling the first tank (2) for effluent to be treated (1);
- starting the first pump (6) in this first tank (2) as soon as a predetermined high level is detected by the level detectors in this first tank (2);
- mixing of the effluent flow emerging from the first tank (2) with a flow of decontamination agent in set proportions;
- the transfer of this mixture into the decontamination tank (8), until a predetermined high-level is detected in this second tank (8) and/or a lack of effluent is detected.

10. Process as described in claim 9, **characterised by** the fact that it involves, in addition, a step for keeping the effluents being treated / decontamination product mixed for an adjustable period of time in the second tank (8).

## Patentansprüche

1. Vorrichtung zur Entgiftung von Abwässern aus medizinischen Analyselaboren oder Krankenhauseinrichtungen, umfassend drei Behälter, nämlich einen ersten Behälter (2) zur Überführung, in weichen die zu behandelnden Abwässer gegossen werden, um dort anschließend in einen zweiten Behälter (8) zur Entgiftung überführt zu werden, in welchem die zu behandelnden Abwässer mit einem entgiftenden Produkt vermischt werden, wobei die beiden Behälter (2,8) mit Niveaudetektoren oder Sonden (3, 4, 5; 13, 16, 17) versehen sind, und einen dritten Vorratsbehälter (9) mit entgiftendem Produkt; wobei die Vorrichtung mit Dosiermitteln (12) versehen ist, die es erlauben, einen vorbestimmten Strom von entgiftendem Produkt in den Ausgußstrom von dem Behälter zur Überführung (2) zu dem Behälter zur Entgiftung (8) zuzuführen,
wobei die Vorrichtung eine zentrale Steuereinheit (7) umfaßt, welche steuert:
- eine erste Pumpe (6) zum Einleiten der Abwässer von dem ersten Behälter (2) in den zweiten Behälter (8);
- eine zweite Pumpe (14) zum Einleiten der behandelten Abwässer, die in dem zweiten Behälter (8) enthalten sind, in den Ausgang zum Abziehen wie einer Abwasserkanalisation oder einer Stelle zur Nachbehandlung;
- Niveaudetektoren (3, 4, 5; 13, 16, 17), welche die Informationen zur zentralen Steuereinheit schicken, welche in Hinblick auf diese Informationen Pumpen (6, 14) und Dosiermittel (12) steuert;
wobei die Vorrichtung **dadurch gekennzeichnet ist, daß** sie die Entgiftung der Abwässer je nach deren Anfall erlaubt und **dadurch**, daß sie außerdem einen Tank umfaßt, der eine Dekompressionskammer bildet, welche geeignet ist, die Kondensation der Nebel sicherzustellen, wie jenen aus Apparaten der Hämatologie, wobei diese kondensierten Nebel anschließend in den ersten Behälter (2) zur Behandlung von Abwässern geführt werden.

2. Vorrichtung nach Anspruch 1, **dadurch gekennzeichnet, daß** sie ein Mittel zum Pumpen des entgiftenden Produktes umfaßt, um es zum Dosiermittel (12) für seine Mischung mit den zu behandelnden Abwässern (1) zu führen.

3. Vorrichtung nach Anspruch 2, **dadurch gekennzeichnet, daß** sie Niveaudetektoren (10, 11) für das entgiftende Produkt umfaßt, das in dem dritten Behälter (9) enthalten ist, wobei diese Niveaudetektoren (10, 11) mit der zentralen Steuereinheit (7) verbunden sind.

4. Vorrichtung nach einem der Ansprüche 1 bis 3, **dadurch gekennzeichnet, daß** die Niveaudetektoren eine obere Niveausonde, eine gemeinsame Sonde und eine untere Niveausonde umfassen, wobei die oberen und unteren Niveausonden für jeden Behälter (2, 8, 9) vorbestimmt sind.

5. Vorrichtung nach einem der Ansprüche 1 bis 4, **dadurch gekennzeichnet, daß** die Pumpen (6, 14) eingetaucht sind.

6. Vorrichtung nach einem der Ansprüche 1 bis 5, **dadurch gekennzeichnet, daß** die Pumpen (6, 14) vom Zentrifugalpumpentyp oder Membranpumpentyp sind.

7. Vorrichtung nach einem der Ansprüche 1 bis 6, **dadurch gekennzeichnet, daß** das Mittel zum Pumpen des entgiftenden Produktes hinter dem dritten Behälter (9) eine Venturipumpe, peristaltische Pumpe oder Membranpumpe umfaßt.

8. Vorrichtung nach einem der Ansprüche 1 bis 7, **dadurch gekennzeichnet, daß** sie Mittel zum manuellen Entleeren der Behälter (2, 8, 9) umfaßt, wie insbesondere Hähne, die im unteren Teil dieser Behälter (2, 8, 9) angeordnet sind.

9. Verfahren zur Verwendung einer Vorrichtung wie in einem der Ansprüche 1 bis 8 gezeigt, **dadurch gekennzeichnet, daß** es umfaßt:
- einen Schritt des Füllens des ersten Behälters (2) mit zu behandelnden Abwässern (1);
- das In-Betrieb-Setzen der Pumpe (6) dieses ersten Behälters (2) sobald ein vorbestimmtes oberes Niveau durch Niveaudetektoren dieses ersten Behälters (2) wahrgenommen wird;
- das Mischen im vorbestimmten Verhältnis von einem Strom von Abwässern des ersten Behälters (2) zu einem Strom von entgiftendem Produkt;
- die Überführung dieses Gemisches in den Behälter zum Entgiften (8) bis zum Detektieren eines vorbestimmten oberen Niveaus in diesem zweiten Behälter (8) und/oder Detektion eines Mangels an Abwässern.

10. Verfahren nach Anspruch 9, **dadurch gekennzeichnet, daß** es außerdem einen Schritt des Aufrechthaltens des Gemisches von in Behandlung befindlichen Abwässern und entgiftendem Produkt für eine regelbare Zeit in dem zweiten Behälter (8) umfaßt.
